Europäisches Patentamt

European Patent Office

Office européen des brevets

(11) Publication number: **0 517 926 A1**

## EUROPEAN PATENT APPLICATION
### published in accordance with Art. 158(3) EPC

(21) Application number: 92901914.9

(22) Date of filing: 27.12.91

(86) International application number:
PCT/JP91/01779

(87) International publication number:
WO 92/12244 (23.07.92 92/19)

(51) Int. Cl.⁵: **C12N 15/10**, C12N 5/16,
//C12P21/02

(30) Priority: 27.12.90 JP 407945/90
27.12.90 JP 407119/90

(43) Date of publication of application:
16.12.92 Bulletin 92/51

(84) Designated Contracting States:
CH DE FR GB LI SE

(71) Applicant: **Japan Tobacco Inc.**
**4-12-62 Higashishinagawa**
**Shinagawa-ku, Tokyo 140(JP)**

(72) Inventor: **HONDA, Zen-ichiro 3-11-5-301,**
**Kohinata**
**Bunkyo-ku**
**Tokyo 112(JP)**
Inventor: **SHIMIZU, Takao 5-9-16-102, Ogikubo**
**Suginami-ku**
**Tokyo 167(JP)**
Inventor: **NAKAMURA, Motonao, Japan**
**Tobacco Inc.Life Science**
**Research Laboratory, 6-2, Umegaoka,**
**Midori-ku,**
**Yokohama-shi, Kanagawa-ken 227(JP)**

(74) Representative: **Reinhard, Skuhra, Weise**
**Postfach 44 01 51 Friedrichstrasse 31**
**W-8000 München 40(DE)**

(54) GENES WHICH CODE FOR GUINEA PIG AND HUMAN PLATELET ACTIVATING FACTOR RECEPTORS.

(57) The base sequence of a gene coding for a platelet activating factor receptor, which is important as a chemical mediator relating to diseases such as allergy or inflammation, has been determined with respect to each of guinea pig and man. Cell transformation has been conducted by using a recombinant DNA wherein genes coding for these receptors were introduced.

Rank Xerox (UK) Business Services

```
ATG GAG TTA AAC AGC TCT TCC CGG GTG GAT TCT GAG TTT CGA TAC ACA CTC TTC CCA ATT
GTT TAC AGC ATT ATC TTT GTG CTG GGG ATC ATC GCC AAT GGC TAT GTG CTA TGG GTC TTT
GCC CGC CTA TAC CCT TCC AAG AAA CTA AAT GAG ATA AAG ATC TTC ATG GTG AAC CTC ACG
GTG GCT GAC CTG CTC TTC CTG ATC ACC TTG CCT CTG TGG ATC GTC TAC TAT TCC AAC CAG
GGC AAC TGG TTT CTA CCC AAA TTC CTT TGT AAC CTG GCT GGC TGC CTC TTC TTC ATC AAC
ACC TAC TGC TCT GTG GCC TTC CTG GGA GTG ATC ACC TAT AAC CGC TTC CAG GCA GTG AAA
TAT CCC ATC AAG ACT GCT CAG GCC ACC ACC CGC AAG CGA GGC ATC GCT TTG TCC TTG GTC
ATC TGG GTG GCC ATT GTG GCT GCT GCA TCT TAC TTC CTT GTC ATG GAC TCC ACC AAC GTA
GTG TCC AAT AAG GCA GGC TCG GGC AAC ATT ACT CGT TGC TTT GAG CAC TAT GAG AAG GGC
AGC AAG CCT GTC CTC ATC ATC CAT AGC TGC ATT GTG CTC GGC TTC TTC ATT GTC TTC CTT
CTC ATC CTC TTC TGC AAC CTG GTC ATC ATC CAC ACG CTG CTC AGG CAG CCA GTG AAG CAG
CAG CGC AAT GCT GAG GTC AGG CGC CGA GCG CTG TGG ATG GTT TGC ACG GTT CTG GCA GTG
TTT GTC ATC TGC TTT GTA CCA CAC CAC ATG GTA CAG CTG CCC TGG ACC CTG GCT GAG CTG
GGG ATG TGG CCA AGC AGC AAC CAC CAG GCT ATT AAT GAT GCA CAT CAG GTC ACC CTC TGC
CTC CTT AGC ACC AAC TGT GTC TTA GAC CCT GTC ATC TAC TGT TTC CTC ACC AAG AAG TTC
CGC AAG CAC CTC ACG CAA AAG CTT AAT ATC ATG CGC AGC AGC CAG AAA TGC TCC CGG GTC
ACC ACT GAC ACA GGC ACT GAA ATG GCC ATT CCA ATC AAC CAC ACT CCT GTC AAT CCC ATC
AAA AAT NNN
```

F I G. 2

Technical Field

The present invention relates to a cDNA for a platelet-activating factor receptor and, more particularly, to cDNAs for a guinea pig platelet-activating factor receptor and a human platelet-activating factor receptor.

Background Art

A platelet-activating factor (to be referred to as a PAF hereinafter) is a new lipid mediator and was recognized as a platelet agglutinating factor derived from a rabbit basophil (Journal of Experimental Medicine Vol. 136, P. 1356, (1972)) and a hypotensive substance existing in a renal medulla (Journal of Clinical Investigation Vol. 39, P. 266 (1960)) for the first time. This PAF is currently receiving a great deal of attention as a chemical mediator associated with pathologic states such as an allergy and an inflammation. Typical physiological activities of this PAF are activation of a platelet, a neutrophil, a monocyte and the like, migration of an eosinophil, blood vessel permeation promotion, blood pressure reduction, broncho-contraction, ischemic necroses of a stomach and a small intestine, and the like. It is assumed that a receptor which specifically recognizes the PAF exists on a cell or tissue, and the PAF is bound to the receptor to cause each of the above activities. Since the PAF is, however, highly hydrophobic, it can easily penetrate into a lipid bilayer. It is, therefore, difficult to conduct a receptor binding experiment and purify the receptor. Although it is currently known that the PAF receptor is conjugated with a GTP-binding protein in information transmission, cloning of a receptor cDNA has not yet been reported. Therefore, it is impossible to form a transformed cell for expressing a PAF receptor at a high rate, so as to allow development of a useful agonist and a useful antagonist, and establishment of a PAF quantitative method.

As described above, since it is very difficult to conduct a binding experiment of the PAF receptor and purify it, the development of effective agonists and antagonists has been delayed. In a future development of agonists and antagonists and clarification of a mechanism for allergies and inflammations, a cDNA for PAF receptor must be determined, and a transformed cell for expressing the PAF receptor at a high rate must be inevitably formed.

Disclosure of Invention

It is an object of the present invention to provide a cDNA for a guinea pig PAF receptor.

It is another object of the present invention to provide cDNA for a human PAF receptor.

On the basis of extensive studies made in consideration of the above situation, the present inventors have determined amino acid sequences of a guinea pig PAF receptor and a human PAF receptor for the first time, and have prepared genes (cDNAs) for coding the amino acid sequences.

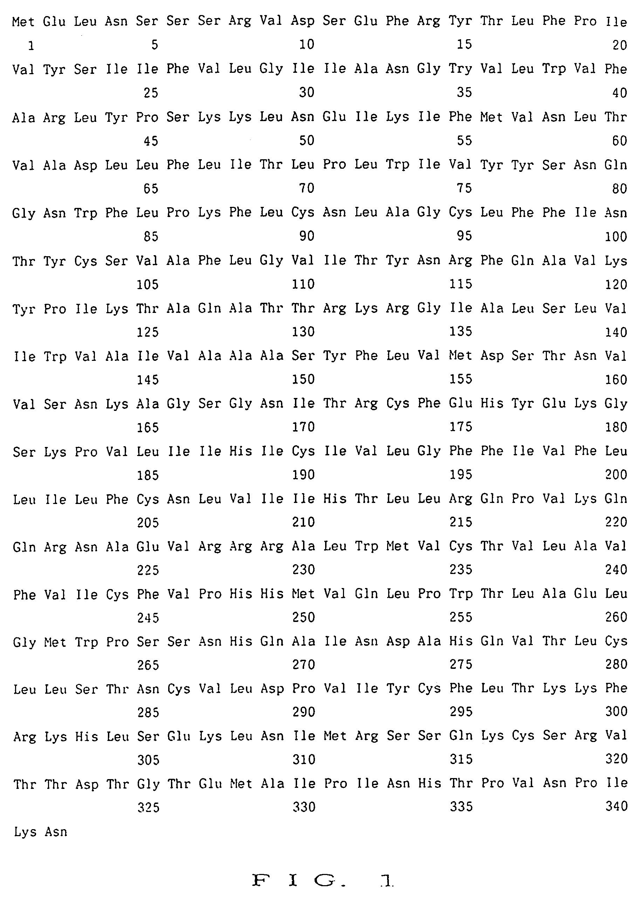

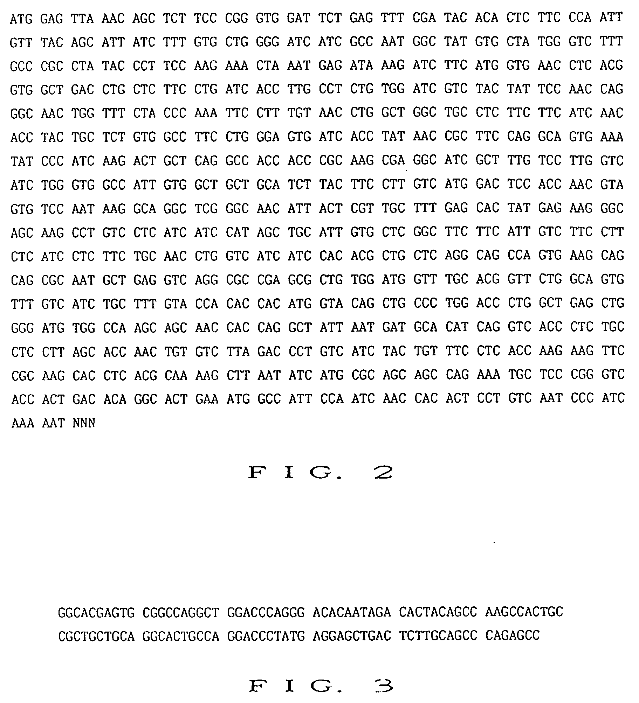

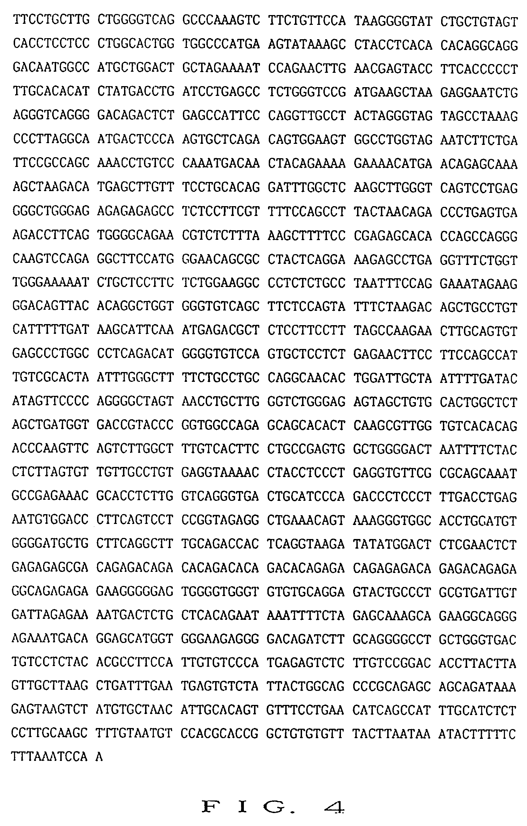

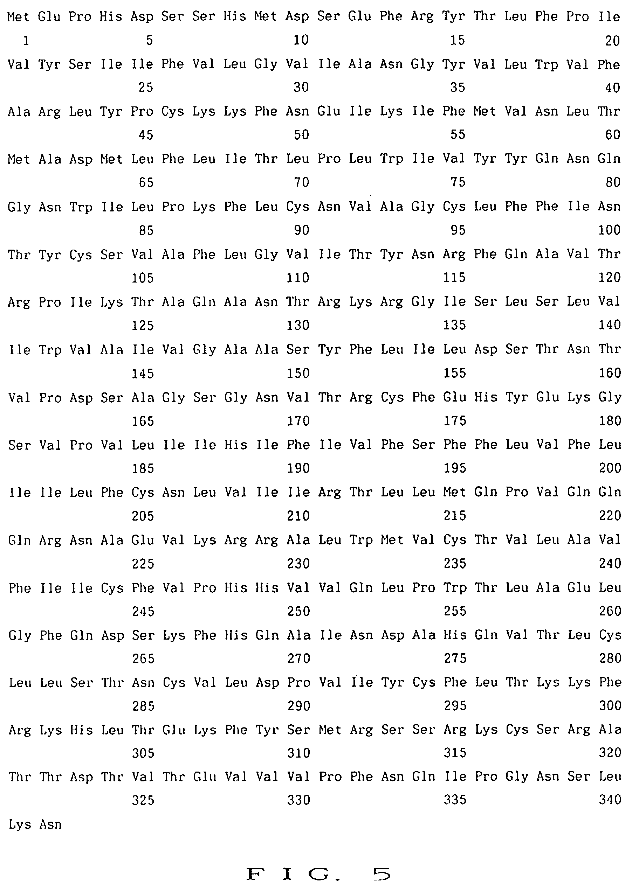

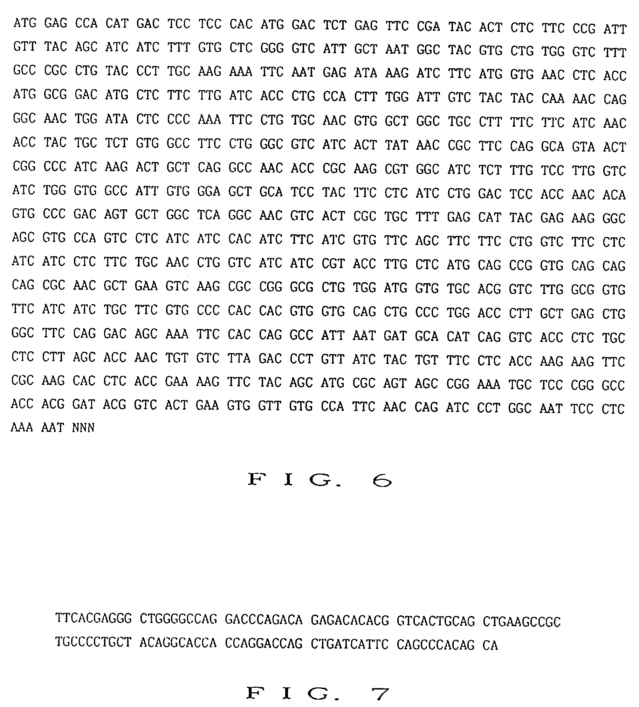

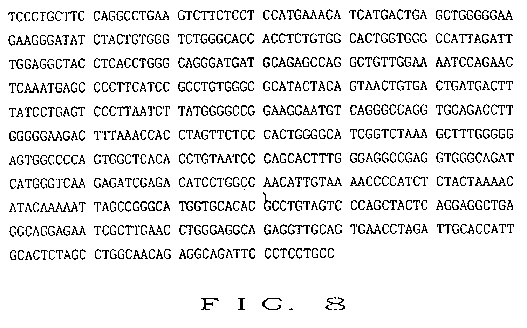

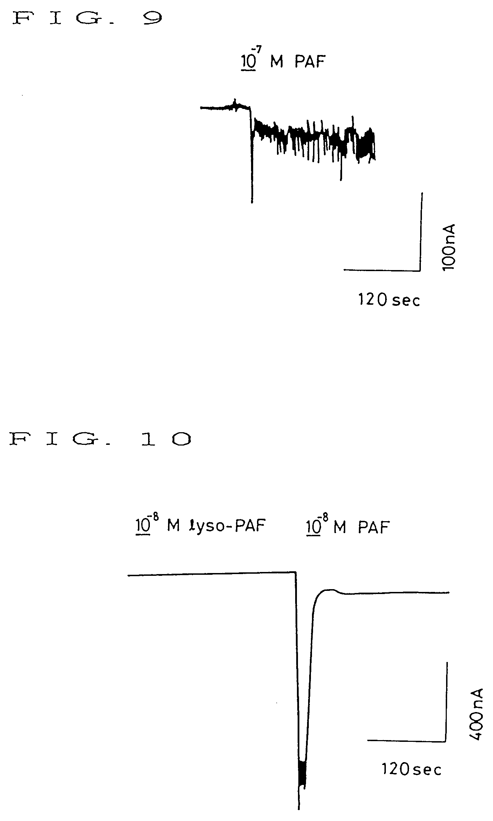

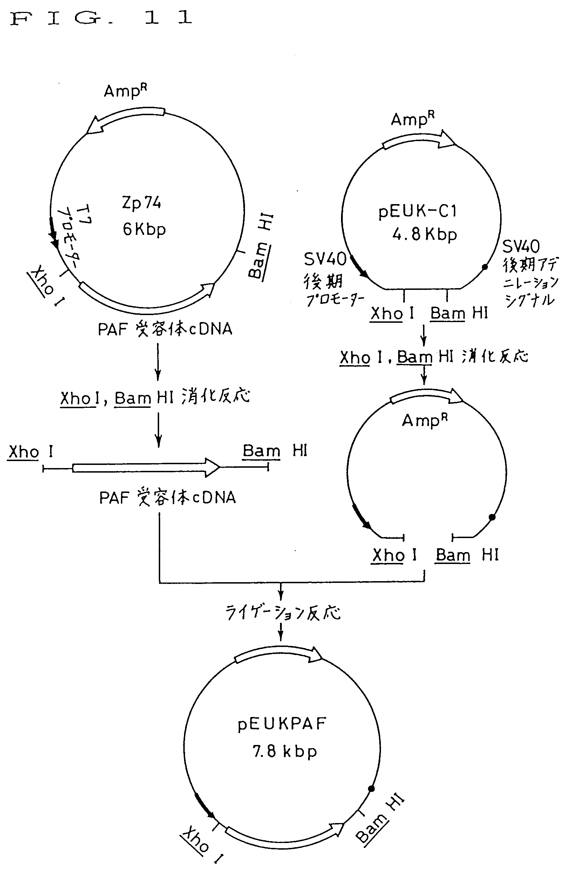

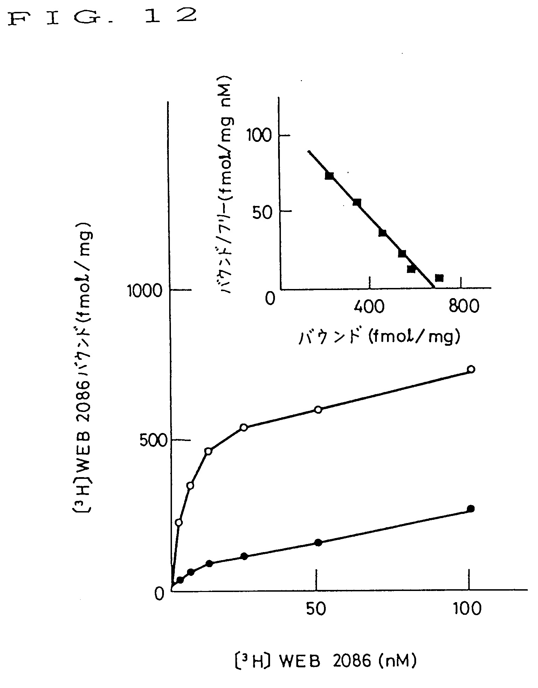

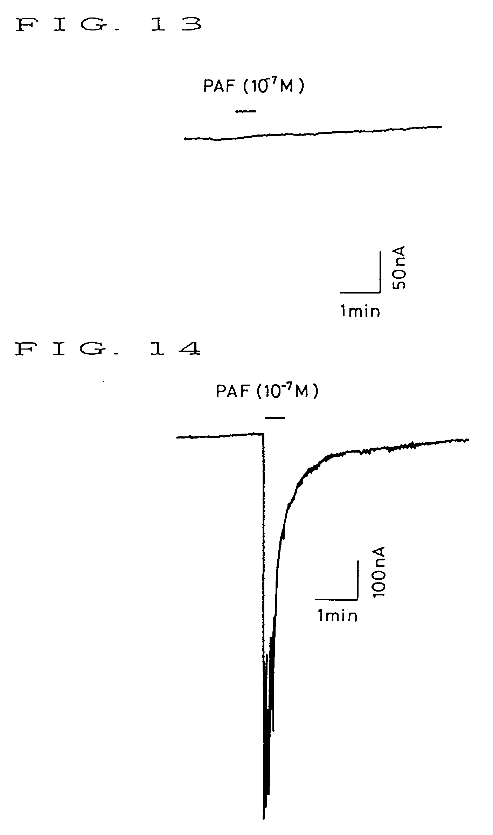

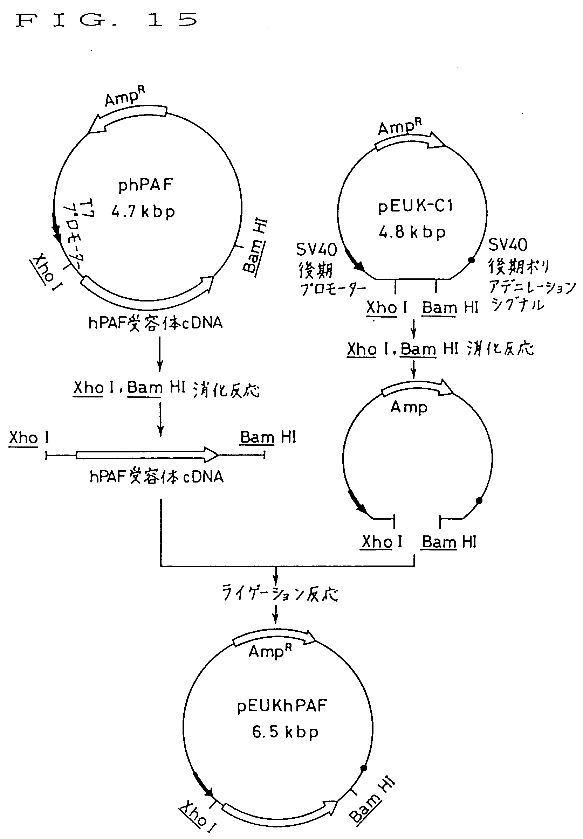

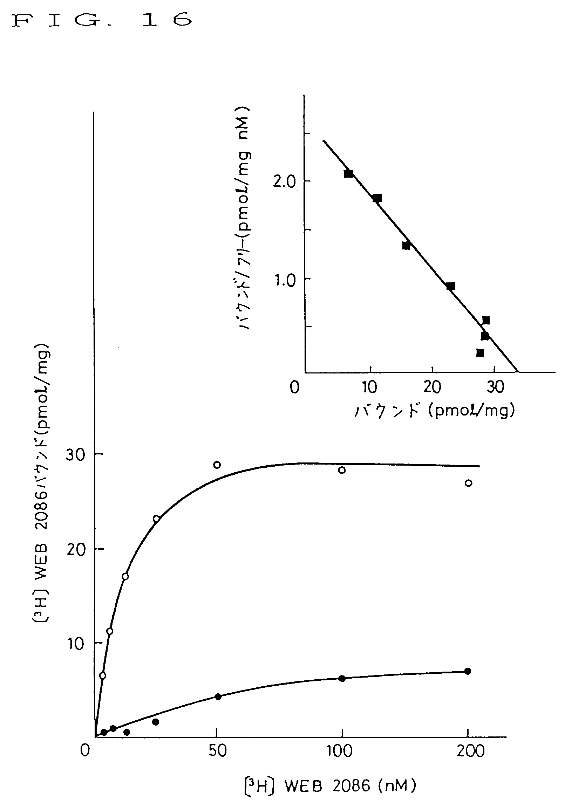

The guinea pig PAF receptor has an amino acid sequence shown in Fig. 1. The gene (cDNA) according to the present invention is a cDNA for this guinea pig PAF receptor and has a base sequence shown in Fig. 2.

As is apparent from the sequence shown in Fig. 2, this cDNA consists of a total of 1029 base pairs having base number 1 to base number 1029. A portion from base number 1 to base number 3 corresponds to an initiation genetic code. A portion from base number 1027 to base number 1029 corresponds to a termination genetic code. Note that NNN from base number 1027 to base number 1029 in the base sequence in Fig. 2 may be any one of TGA, TAA, and TAG. The initiation code and the termination code can be linked to arbitrary base pairs, respectively. The types and number of base pairs are not important. The base pair need only be an appropriate base pair to be linked to an extranuclear gene of bacteria or the like. For example, the initiation code can be linked to base pairs constituting a base sequence shown in Fig. 3, and the termination code can be linked to base pairs constituting a base sequence shown in Fig. 4.

The human PAF receptor has an amino acid sequence shown in Fig. 5. Another gene (cDNA) according to the present invention is a gene for coding the human PAF receptor and has a base sequence shown in Fig. 6. As is apparent from the sequence shown in Fig. 6, this cDNA consists of a total of 1029 base pairs having base number 1 to base number 1029. A portion from base number 1 to base number 3 corresponds to an initiation genetic code. A portion from base number 1027 to base number 1029 corresponds to a termination genetic code. Note that NNN from base number 1027 to base number 1029 in the base sequence in Fig. 6 may be any one of TGA, TAA, and TAG. The initiation code and the termination code can be linked to arbitrary base pairs, respectively. The types and number of base pairs are not important. The base pair need only be an appropriate base pair to be linked to an extranuclear gene of bacteria or the like. For example, the initiation code can be linked to base pairs constituting a base sequence shown in Fig. 7, and the termination code can be linked to base pairs constituting a base sequence shown in Fig. 8.

The cDNA for the guinea pig PAF receptor can be obtained by a method of utilizing a gene expression system using, e.g., Xenopus oocyte from a cDNA library derived from a guinea pig lung mRNA and electrophysiologically detecting the expression of the receptor cDNA. More specifically, the cDNA in the library is used as a template to perform in-vitro synthesis of an RNA, and the synthesized RNA is micro-injected into the oocyte and is translated therein. If an RNA for the target PAF receptor is present in the micro-injected RNA, the oocyte expresses the PAF receptor. Upon contact with the PAF, signalling is transmitted in an order of (conjugating between the PAF receptor and the GTP-binding protein) → - (activation of phospholipase C) → (ascending of $IP_3$) → (ascending of $Ca^{2+}$). Finally, a $C\ell$- channel is opened.

The opening of the channel is detected in a change of membrane current by an electrophysiological technique (voltage clamp method). For example, if the above response indicating the presence of the PAF receptor cDNA is obtained in the $6 \times 10^4$ cDNAs pool as a template from the phage library, this is sequentially divided to repeat the assay, thereby finally obtaining one target clone.

The resultant cDNA is inserted in an appropriate vector (e.g., plasmid or phage), and then the vector having the cDNA inserted is introduced in bacteria serving as a host, thereby mass-producing the PAF receptor.

The cDNA library for obtaining a cDNA for the guinea pig PAF receptor according to the present invention can be any library if it is derived from guinea pig and an internal organ having a PAF receptor binding activity. For example, a guinea pig leucocyte cDNA library or a guinea pig renal cDNA library can be used.

As described above, a cDNA having all genetic information required to form a guinea pig PAF receptor according to the present invention can be obtained.

The cDNA for the human PAF receptor according to the present invention can be obtained from a cDNA library derived from a human leucocyte mRNA by utilizing, e.g., a probe hybridization method. According to this method, by using the guinea pig PAF receptor cDNA having a known base sequence as a probe, a DNA having a base sequence complementary with that of the probe can be obtained from the cDNA library.

The resultant cDNA is inserted in an appropriate vector (e.g., plasmid or phage), and then the vector having the cDNA inserted is introduced in bacteria serving as a host, thereby mass-producing the PAF receptor.

The cDNA library for obtaining a cDNA for the human PAF receptor according to the present invention can be any library if it is derived from human and an internal organ having a PAF receptor binding activity. For example, a human lung cDNA library or a human renal cDNA library can be used.

As described above, a cDNA having all genetic information required to form a human PAF receptor according to the present invention can be obtained.

Brief Description of Drawings

Fig. 1 is a view showing an amino acid sequence of a guinea pig PAF receptor;

Fig. 2 is a view showing a base sequence of a cDNA for the guinea pig PAF receptor according to the present invention;

Fig. 3 is a view showing a base sequence of base pairs capable of linking to an initiation code portion of the cDNA for the guinea pig PAF receptor according to the present invention;

Fig. 4 is a view showing a base sequence of base pairs capable of linking to a termination code portion of the cDNA for the guinea pig PAF receptor according to the present invention;

Fig. 5 is a view showing an amino acid sequence of a human PAF receptor;

Fig. 6 is a view showing a base sequence of a cDNA for the human PAF receptor according to the present invention;

Fig. 7 is a view showing a base sequence of base pairs capable of linking to an initiation code portion of the cDNA for the human PAF receptor according to the present invention;

Fig. 8 is a view showing a base sequence of base pairs capable of linking to a termination code portion of the cDNA for the human PAF receptor according to the present invention;

Fig. 9 is a view showing measurement results according to a voltage clamp method for measuring expression obtained when an RNA containing a guinea pig PAF receptor cDNA derived from $6 \times 10^4$ phages is micro-injected into Xenopus oocyte;

Fig. 10 is a view showing measurement results according to the voltage clamp method for measuring expression obtained when an a cRNA synthesized using a single guinea pig PAF receptor cDNA as a template is micro-injected into Xenopus oocyte;

Fig. 11 is a view showing the process for constructing a COS expression plasmid pEUKPAF;

Fig. 12 is a view showing PAF receptor binding activity data in the COS7 cell in which the plasmid pEUKPAF is introduced;

Fig. 13 is a view showing measurement results obtained when an Xenopus oocyte micro-injected with water is measured according to the voltage clamp method;

Fig. 14 is a view showing measurement results according to the voltage clamp method for measuring expression obtained when an RNA synthesized using a cloned human PAF receptor cDNA as a template is microinjected into Xenopus oocyte;

Fig. 15 is a view showing the process for constructing a COS 7 intracellular expression plasmid pUEKhPAF; and

Fig. 16 is a graph showing PAF receptor binding activity in the COS 7 cell to which the plasmid pEUKhPAF is introduced.

Best Mode of Carrying Out the Invention

The present invention will be described in detail by way of its examples. It is apparent that the present invention is not limited to the following examples. In the following examples, the operations are performed in accordance with the method described in Molecular Cloning Laboratory Manual, Maniatis, et al., 2nd ed. (1989), Cold Spring Harbor, unless otherwise specified. Restriction enzymes are commercially available ones and are practically applied in accordance with the attached instructions.

Example 1

In this example, a method of manufacturing a DNA having a base sequence obtained by binding the base pairs having the sequence of Fig. 3 to the side of base number 1 of the gene having the base sequence shown in Fig. 2, and by binding the base pairs having the sequence of Fig. 4 to the side of base number 1029 thereof and a method of manufacturing a transformant using the resultant DNA will be described below.

(1) Preparation of Guinea Pig Lung mRNA

Lungs (about 5g) were enucleated from two female Hartley guinea pigs (about 350g each) and were immediately frozen to prevent decomposition of the mRNA. These lungs were pulverized with a hammer while cooling them with liquid nitrogen, and 30 mℓ of a GTC buffer solution (6M guanidinethiocyanate, 5 mM solidum citrate, 17 mM sodium sarcosile, and 0.7% $\beta$-mercaptoethanol) were added thereto to homogenize the pulverized lungs. The resultant homogenate was centrifuged at room temperature at 3,000 × g for 5 minutes, and the supernatant was transferred to another tube. 25 mℓ of the supernatant of the homogenate were layered on a cushion (8 mℓ) of 5.7 M cesium chloride containing 0.1 M EDTA and were centrifuged by using SRP28 Rotor available from Hitachi, Ltd. at 15°C and 26,000 × g for 65 hours. After this centrifugation, the brown and dark brown protein layers, and the DNA layer were eliminated by an aspirator. The RNA precipitated on the bottom of the tube was dissolved in 1 mℓ of a TES buffer solution (100 mM tris-HCℓ, 5 mM EDTA, and 1% SDS; pH 7.5) and was extracted from phenol-chloroform (1 : 1). 0.1 mℓ of 3M sodium acetate and 2 mℓ of cooled ethanol were added to the aqueous layer containing the RNA to precipitate the RNA. The RNA recovered by centrifugal separation was washed with 80% ethanol and was dried in a vacuum. The dried RNA was dissolved in sterilized distilled water.

The resultant aqueous RNA solution was passed through an oligo (dT) cellulose column to separate an mRNA having a poly (A). More specifically, a balanced buffer solution (0.5 M NaCℓ, 20 mM tris-HCℓ, 1 mM EDTA, and 0.1% SDS; pH 7.6) of a 2-fold volume was added to the RNA dissolved in the sterilized distilled water, and the resultant mixture was heated at 65°C for 5 minutes. The incubated solution was passed through an oligo (dT) cellulose column balanced by a balanced buffer solution in advance to wash the column with a balanced buffer solution of a 10-fold volume. After this washing, the mRNA was eluted by an elution buffer solution (20 mM tris-HCℓ and 1 mM EDTA; pH 7.6). The eluted mRNA was recovered by ethanol precipitation. The recovered mRNA was washed with 80% ethanol and dried in a vacuum. The dried mRNA was dissolved in sterilized distilled water.

The mRNA was subjected to size fractionation by a 5 to 30% sucrose gradient centrifugation according to the following procedures. About 150 μg of the RNA heated at 65°C for 5 minutes was layered on a solution (10 mM tris-HCℓ, 1 mM EDTA, and 0.1% SDS; pH 7.5) having a 5 to 30% sucrose density gradient and was centrifuged using an RPS40T Rotor available from Hitachi, Ltd. at 15°C and 24,000 × g for 18 hours. After the centrifugation, the resultant solution was fractionated in units of 0.4 mℓ. Each of these fractions was subjected to ethanol precipitation to recover the RNA. A portion of each fraction was subjected to electrophoresis in a 1% agarose gel to identify a fraction containing a 18SrRNA. By using fractions containing RNA having molecular weights larger than that of the 18SrRNA, the following cDNA synthesis was performed.

(2) Preparation of cDNA Library

cDNAs were synthesized from the prepared mRNA using a cDNA synthesis system commercially available from Pharmacia, Inc. in accordance with the protocol attached to the product. More specifically, cDNAs complementary with the mRNA were synthesized by a reverse transcriptase, and then a nick and a gap were formed in an RNA-strand by ribonuclease H. Repair synthesis was performed using T4DNA polymerase to substitute the RNA-strand with the cDNA-strand. Finally, both cohesive ends of the cDNA-strand were converted into flush ends by Klenow fragments of E.coli DNA polymerase I. A cDNA library was prepared using a λZAPII library system available from Stratagene Corp. by a method following the protocol attached to the product. More specifically, EcoRI adapters (without any NotI site) were added to both the ends of the synthesized cDNA, and the resultant structure was bound to the λZAPII arm cleaved by the EcoRI in advance. Thereafter, in-vitro packaging was performed, and the resultant phage was infected to an E.coli XLI-Blue strain (attached to the product) to prepare the cDNA library.

(3) Selection of cDNA Clone for Coding PAF Receptor

The same method as cloning of a substance K receptor cDNA (Nature, Vol. 329, P. 836 (1987)) and a serotonin 1c receptor cDNA (Science, Vol. 241, P. 558, (1988)), i.e., a method utilizing a gene expression system of Xenopus oocyte was employed in screening of a PAF receptor cDNA from the cDNA library.

The library was divided into phage groups each consisting of $6 \times 10^4$ clones, and a phage DNA was prepared by the conventional procedures, i.e., polyethyleneglycol precipitation, a phenol-chloroform (1 : 1) treatment, and several times of ethanol precipitation. The NotI site located in the downstream of the T7 promoter was cleaved in this phage DNA (about 10 μg) and was used as a template for in-vitro RNA synthesis. The RNA synthesis was performed by reacting 10 units of T7RNA polymerase at 37°C for 60 minutes in a reaction solution containing 40 mM tris-HCℓ (pH 7.9), 100 μg/mℓ BSA, 10 mM DTT, 6 mM MgCℓ$_2$, 2 mM spermidine, and 1 μg/μℓ human placenta ribonuclease inhibitor in the presence of 0.5 mM Cap-analogue 7-methyl-GpppG, 0.5 mM ATP, 0.5 mM UTP, 0.5 mM CTP, and 0.05 mM GTP. After this reaction, a phenol-chloroform (1 : 1) treatment and ethanol precipitation were performed to recover the synthesized RNA. Finally, the RNA was dissolved in the sterilized water in a concentration of 1 μg/μℓ.

Oocytes for micro-injecting the synthesized RNA were prepared as follows. The belly of a Xenopus (purchased from Hamamatsu Laborator Animal Supplier) narcotized in ice was cut with scissors by about 5 mm, and 1 to 2g of an aggregate of oocytes was taken out with tweezers. This aggregate was immediately placed in a small Petri dish containing 3 mℓ of an MBS (88 mM NaCℓ, 1 mM KCℓ, 2.4 mM NaHCO$_3$, 15 mM tris-HCℓ, 0.3 mM Ca(NO$_3$)$_2$·4H$_2$O, 0.41 mM CaCℓ$_2$·4H$_2$O, 0.82 mM MgSO$_4$·7H$_2$O, 10 μg/mℓ penicillin sodium salt, and 10 μg/mℓ streptomycin sulfate; pH 7.6). The aggregate was washed with the MBS three times and was treated in the MBS containing 0.2% (w/v) collagenase type II (available from Sigma Corp.) at room temperature for 1.5 hours. After the treatment, the aggregate of oocytes was washed with fresh MBS four or five times. Follicular cell membranes were removed from the oocyte surfaces with tweezers under the observation with a stereoscopic microscope. 50 nℓ of the synthesized RNA were micro-injected into each oocyte from which the follicular cell membrane was removed. Thereafter, the oocytes were cultured in the MBS at 20°C for three days, thereby synthesizing a protein derived from the injected RNA.

Whether the PAF receptor was contained in the protein synthesized in each oocyte was determined using a Ca$^{2+}$-sensitive Cℓ$^-$ channel opening having ligand dependency as an index. This could be detected by measuring a change in membrane potential using the voltage clamp method.

A 0.5 mℓ tank was set under the stereoscopic microscope, and oocytes were placed while a Ringer's solution (115 mM NaCℓ, 2 mM KCℓ, 5 mM HEPES, 1.8 mM CaCℓ$_2$, and 1 mg/mℓ BSA; pH 7.2) was perfused in the tank and then the memberance potential of the oocytes were clamped. In this case, the command potential was set to be a reversal potential (normally -50 to -100 mV) or less of Cℓ$^-$ in the Ringer's solution. While the membrane potential of the oocytes were kept clamped, the Ringer's solution containing a $10^{-7}$ M PAF was flowed. In an oocyte on which the PAF receptor was expressed, the PAF was bound to the receptor. Thus, a series of reactions occurred in an order of (activation of the GTP-binding protein) → (activation of phospholipase C) → (ascending of intracellular $Ca^{2+}$) → (opening of $C\ell^{-}$ channel), and an outward clamp current was obtained as a signal. This signal was obtained from one phage group when five groups each consisting of $6 \times 10^4$ clones were screened. This response is shown in Fig. 9. As shown in Fig. 9, a membrane current having a typical two-phase time course inherent to a receptor associated with the GTP-binding protein was observed. While the response was observed, the phage group of $6 \times 10^4$ clones was sequentially divided. Finally, a single clone could be obtained after seven cycles. Fig. 10 shows the response obtained when the synthesized RNA obtained by using the single clone as a template was micro-injected.

Plasmid (Zp74) containing inserts were recovered by a plasmid rescue method using an f1 helper phage R408 (available from Stratagene Corp.) from the resultant phage clone. In this case, the details of the method entirely followed the protocol provided by Stratagene Corp. The length of the cDNA insert in Zp74 was about 3 Kbp.

### (4) Determination of Complete Base Sequences of cDNA Insert

The complete base sequence of the cDNA insert was determined using a deletion kit for kilo sequence (purchased from TAKARA SYUZO CO., LTD.) and a sequenase DNA sequence kit (purchased from TOYOBO CO., LTD.). The complete base sequence determined are shown in Figs. 2, 3, and 4. The sequence shown in Fig. 3 is bound to a base, which has base number 1, of the sequence shown in Fig. 2, and the sequence shown in Fig. 4 is bound to a base, which has base number 1029, of the sequence shown in Fig. 2. Clone Zp74 contains 3017 base pairs as inserted DNA and has 1029 base pairs from the initiation genetic code ATG to the termination genetic code TAG as a largest continuous open leading frame. An amino acid sequence derived from the DNA base sequence shown in Fig. 2 is shown in Fig. 1.

### (5) Construction of Expression Plasmid pEUKPAF for COS Expression of Guinea Pig PAF Receptor cDNA

An expression plasmid shown in Fig. 11 was constructed so as to introduce the cloned guinea pig PAF receptor cDNA into COS cells and to examine the presence/absence of expression.

A COS expression vector pEUK-C1 (available from Clonetec Corp.) was purchased from TOYOBO CO., LTD. This vector has SV 40 late promoter for intiating a transcription of an introduced cDNA and SV40 late polyadenilation signal and also has five cloning sites therebetween.

5 μg of the plasmid Zp74 was digested by 5 units of BamHI and 5 units of XhoI, and the resultant reaction product was separated by electrophoresis using 1% agarose gel. About 3 Kbp of a DNA fragment containing the RAF receptor cDNA in the agarose gel was recovered using Gene clean (available from Biotec Corp.) in accordance with the attached protocol. The recovered DNA fragment was subjected to ethanol precipitation and was then dissolved in sterilized water.

5 μg of the expression vector pEUK-C1 was digested by 5 units of BamHI and 5 units of XhoI and then about 4.8 Kbp of a DNA fragment was recovered following the same procedure described above. The recovered DNA fragment was dissolved in sterilized water.

The two recovered DNA fragments, i.e., about 200 ng of the DNA fragment containing the PAF receptor cDNA and about 50 ng of the vector DNA fragment were mixed and reacted to be bound. This reaction was performed using a TAKARA ligation kit in accordance with the attached protocol. By using this reaction solution, E.coli XL1-Blue was transformed by a conventional method to obtain a colony having resistance to Ampicillin ($Amp^R$).The plasmid DNA was recovered from this colony. The recovered plasmid was confirmed to be a target plasmid by agarose gel electrophoresis after this plasmid was digested by BamHI and XhoI. As described above, COS expression plasmid pEUKPAF was constructed.

### (6) Expression of Guinea Pig PAF Receptor cDNA in COS7 Cells

COS expression plasmid pEUKPAF constructed in (5) was introduced into a COS7 cell as a kind of a COS cell to examine the presence/absence of expression of the PAF receptor cDNA. The experiment was conducted in the following three steps.

### (a) Introduction of Plasmid pEUKPAF into COS7 Cells

COS 7 cells were cultured in a DMEM medium (available from Nissui Seiyaku KK) added with a 20% bovine fetal serum (available from Boehringer Mannheim Corp.), 0.6 g/ℓ L-glutamine, 1.5 g/ℓ sodium hydrogenecarbonate, 100 units/mℓ penicillin, and 50 fg/mℓ streptomycin at 37°C and in a condition of 5%

$CO_2$. When the plasmid was introduced, the number of cells was adjusted in the order of $10^7$ cells/m$\ell$. A Select transfection kit available from Pharmacia Inc. was used to introduce the plasmid in accordance with the attached protocol. The cells introduced with the plasmid were kept cultured for three days to wait for expression of the introduced cDNA.

(b) Preparation of Membrane Fraction From Transformed Cells

After the culture, the transformed cells attached to the bottom surface of the culture vessel were recovered by sufficiently suspending the cells and centrifuging it at 800 × g and 4°C for 10 minutes. The precipitated cells were suspended in an HEPES buffer solution (25 mM HEPES, 10 mM MgC$\ell_2$, and 0.25 M sucrose; pH 7.4) cooled with ice, and were perfectly crushed by homogenizing the suspension on ice.

The resultant solution was centrifuged at 800 × g and 4°C for 10 minutes to obtain a supernatant, the supernatant was further centrifuged at 40,000 × g and 4°C for 20 minutes to obtain a precipitate, and the resultant precipitate was suspended in an HEPES buffer solution to obtain a membrane fraction subjected to a PAF receptor binding experiment.

(c) Confirmation of PAF Receptor Expressed on COS7 Cells

Whether the PAF receptor was expressed on the transformed cell was confirmed by checking the presence/absence of PAF receptor binding activity in the prepared membrane fraction. This experiment was conducted using PAF antagonist WEB2086 (available from NEN Corp.) labelled with tritium. To 100 $\mu\ell$ of a reaction buffer solution (25 mM HEPES, 10 mM MgC$\ell_2$, and 0.1% BSA; pH 7.4), 100 nM, 50 nM, 25 nM, 12.5 nM, or 6.25 nM [$^3$H] WEB2086 and 100 $\mu\ell$ of the membrane fraction prepared in the step (b) were added and the resultant mixture was reacted at 25°C for 90 minutes. Immediately after this reaction, the reaction solution was filtered through a glass filter (available from Wattman Corp.; GF/C) to cause the glass filter to adsorb the membrane fraction. The glass filter was sufficiently washed with a reaction buffer solution to remove a 2on-adsorbed [$^3$H] WEB2086. Thereafter, this glass filter was dried at 80°C for 30 minutes, and then added with liquid scintillator to measure a count. Note that a binding count in the presence of 10 $\mu$M PAF in a reaction system at each point was also measured in order to detect nonspecific adsorption of the [$^3$H] WEB2086 to the membrane. Fig. 12 shows the result. Referring to Fig. 12, a specific bond is represented by o, and a non-specific bond is represented by •. It was therefore confirmed that the PAF receptor was expressed on the transformed cell membrane.

Example 2

In this example, a method of manufacturing a DNA having a base sequence obtained by binding the base pairs having the sequence of Fig. 7 to the side of base number 1 of the cDNA having the base sequence shown in Fig. 6 and the base pairs having the sequence of Fig. 8 to the side of base number 1029 thereof, and a method of manufacturing a transformant using the resultant DNA will be described below.

(1) Preparation of Human Leukocyte mRNA

Human leukocytes previously frozen and preserved at -80°C were added with 30 m$\ell$ of a GTC buffer solution (6M guanidinethiocyanate, 5 mM solidum citrate, 17 mM sodium sarcosile, and 0.7% $\beta$-mercaptoethanol) and then homogenized quickly.

The resultant homogenate was centrifuged at room temperature at 3,000 × g for 5 minutes, and the supernatant was transferred to another tube. 25 m$\ell$ of the supernatant of the homogenate were layered on a cushion (8 m$\ell$) of 5.7 M cesium chloride containing 0.1 M EDTA and were centrifuged by using SRP28 Rotor available from Hitachi, Ltd. at 15°C and 26,000 × g for 65 hours. After this centrifugation, the brown and dark brown protein layers, and the DNA layer were eliminated by an aspirator. The RNA precipitated on the bottom of the tube was dissolved in 1 m$\ell$ of a TES buffer solution (100 mM tris-HC$\ell$, 5 mM EDTA, and 1% SDS; pH 7.5) and was extracted from phenol-chloroform (1 : 1).

0.1 m$\ell$ of 3M sodium acetate and 2 m$\ell$ of cooled ethanol were added to the aqueous layer containing the RNA to precipitate the RNA. The RNA recovered by centrifugal separation was washed with 80% ethanol and was dried in a vacuum. The dried RNA was dissolved in sterilized water. The resultant aqueous RNA solution was passed through an oligo (dT) cellulose column to separate an mRNA having a poly (A). More specifically, a balanced buffer solution (0.5 M NaC$\ell$, 20 mM tris-HC$\ell$, 1 mM EDTA, and 0.1% SDS;

pH 7.6) of a 2-fold volume was added to the RNA dissolved in the sterilized water, and the resultant mixture was heated at 65°C for 5 minutes. The heat-treated solution was passed through an oligo (dT) cellulose column balanced by a balanced buffer solution in advance to wash the column with a balanced buffer solution of a 10-fold volume. After this washing, the mRNA was eluted by an elution buffer solution (20 mM tris-HCℓ and 1 mM EDTA; pH 7.6). The eluted mRNA was recovered by ethanol precipitation. The recovered mRNA was washed with 80% ethanol and dried in a vacuum. The dried mRNA was dissolved in sterilized water. cDNAs were synthesized using 3 μg of this mRNA in the following manner.

(2) Preparation of cDNA Library

cDNAs were synthesized from the prepared mRNA using a cDNA synthesis system commercially available from Pharmacia, Inc. in accordance with the protocol attached to the product. More specifically, cDNAs complementary with the mRNA were synthesized by a reverse transcriptase, and then a nick and a gap were formed in an RNA-strand by ribonuclease H. Repair synthesis was performed using T4DNA polymerase to substitute the RNA-strand with the cDNA-strand. Finally, both cohesive ends of the cDNA-strand were converted into flush ends by Klenow fragments of E.coli DNA polymerase I. A cDNA library was prepared using a λZAPII library system available from Stratagene Corp. by a method following the protocol attached to the product. More specifically, EcoRI adapters (without any NotI site) were bound to both the ends of the synthesized cDNA, and the resultant structure was bound to the λZAPII arm cleaved with the EcoRI in advance. Thereafter, in-vitro packaging was performed, and the resultant phage was infected to an E.coli XLI-Blue strain (attached to the product) to prepare the cDNA library.

(3) Selection of cDNA Clone for Human PAF Receptor

A phage group containing about 300,000 phages was selected from the cDNA library prepared in (2) described above and was absorbed in an E.coli XL1-Blue strain, placed on an agar plate, and cultured at 37°C for 12 hours. A nitrocellulose filter was placed on the agar to adsorb the phages and was removed from the agar, and a denaturation treatment was performed in a denaturation solution (0.2 N NaOH and 1.5 M NaCℓ) for five minutes. After the denaturation solution was sufficiently removed with a paper towel, a neutralizing reaction was performed in a neutralizing solution (1.5 M tris-HCℓ and 1.5 M NaCℓ; pH 7.5) for five minutes. Washing was then performed in a 2x SSC (wherein 1x means a 15 mM sodium citrate buffer solution containing 0.15 M sodium chloride; pH 7.0) for 5 minutes. The filter was then dried and heated at 80°C for 2 hours. The filter was then cooled to room temperature and was reacted in a solution obtained by mixing 5x Denhardt solution (wherein 1x represents 0.02% Ficoll, 0.02% polyvinylpyrrolidone, and 0.02% bovine serum albumin), a NET solution (5 M NaCℓ, 2 M tris-HCℓ, and 0.5 M EDTA; pH 8.0), 0.1% sodium dodecyl sulfate, and 100 μg/mℓ denatured salmon sperm DNA at 55°C for 16 hours.

The probe was labelled with $^{32}$P by a multiplimer kit (Amersham Corp.) using an SmaI-digested fragment (about 800 bp) of the guinea pig PAF receptor cDNA as a template. The probe labelled with $^{32}$P was added to the above reaction solution, and a hybridization reaction was performed for 24 hours. Thereafter, the filter was washed twice with 300 mℓ of 2x SSC containing 0.1% sodium dodecyl sulfate at room temperature for 5 minutes each. In addition, the filter was further washed twice with 300 mℓ of 2 × SSC containing 0.1% sodium dodecyl sulfate at 55°C for 20 minutes each. Thereafter, the filter was dried, and autoradiography was performed using an intensifying screen at -70°C for two days.

A DNA probe labelled with $^{32}$P was bound to the phage DNA in which the cDNA of a cDNA for the human PAF receptor was inserted. Therefore, a portion corresponding to a plaque of the phage DNA adsorbed on the nitrocellulose filter appeared as a black dot on an X-ray film. The phage of the plaque appearing as the black dot was separated singly from the agar plate, thereby obtaining a clone in which a cDNA having a length of about 1.7 Kbp was inserted.

A plasmid (phPAF) containing an insert was recovered from the resultant phage clone in accordance with a plasmid rescue method using f1 helper phage R408 (available from Strategene Corp.) In this case, the details of the method followed the protocol provided by Stratagene Corp.

(4) Determination of Complete Base Sequence of cDNA Insert

The complete base sequence of the cDNA insert was determined using a deletion kit for a kilo sequence (purchased from TAKARA SYUZO CO., LTD.) and a sequenase DNA sequence kit (purchased from TOYOBO CO., LTD.). The complete base sequence determined is shown in Figs. 6, 7, and 8. The sequence shown in Fig. 7 is bound to a base of base number 1 of the sequence shown in Fig. 6, and the sequence shown in Fig. 8 is bound to a base of base number 1029 of the sequence shown in Fig. 6. Clone phPAF contains 1780 base pairs as inserted DNA and has 1029 base pairs from the initiation genetic code ATG to the termination genetic code TAG as a largest continuous open leading frame. An amino acid sequence derived from the DNA base sequence shown in Fig. 6 is shown in Fig. 5.

(5) Expression of Human PAF Receptor cDNA in Xenopus Oocyte

It was confirmed utilizing a gene expression system of a Xenopus oocyte that the cloned DNA coded for the human PAF receptor. The experiment was conducted in the following three steps.

(a) In-Vitro RNA Synthesis Using Cloned DNA as Template

5 $\mu$g of plasmid phPAF was digested by restriction enzyme NotI, and subjected to phenol-chloroform (1 : 1) extraction and ethanol precipitation. The resultant product was dissolved in 50 $\mu\ell$ of sterilized water. This DNA solution was mixed with 50 $\mu\ell$ of a reaction buffer solution (1 mM Cap-analogue 7-methyl-GpppG, 1 mM ATP, 1 mM CTP, 1 mM UTP, 0.1 mM GTP, 80 mM tris-HC$\ell$ (pH 7.9), 200 fg/m$\ell$ BSA, 20 mM DTT, 12 mM MgC$\ell_2$, 4 mM spermidine, and 2 $\mu$g/$\mu\ell$ human fetal ribonuclease inhibitor), and 10 units of T7RNA polymerase was added thereto to perform a reaction at 37°C for 60 minutes to synthesize the RNA. After the reaction, 5 units of deoxyribonuclease was added thereto, and the mixture was incubated at 37°C for 15 minutes to decompose a template DNA. The resultant reaction solution was subjected to phenol-chloroform extraction and ethanol precipitation to recover the RNA. The recovered RNA was dissolved in sterilized water at a concentration of 1 $\mu$g/$\mu\ell$.

(b) Preparation of Oocytes into which Synthesized cRNA is microinjected

A female Xenopus (about 100g) used in the experiment was purchased from the Hamamatsu Laboratory Animal Supplier. The belly of a Xenopus narcotized in ice was cut with scissors by about 5 mm, and 1 to 2g of an aggregate of oocytes was taken out with tweezers. This aggregate was immediately placed in a small Petri dish containing 3 m$\ell$ of an MBS (88 mM NaC$\ell$, 1 mM KC$\ell$, 2.4 mM NaHCO$_3$, 15 mM tris-HC$\ell$, 0.3 mM Ca(NO$_3$)$_2$·4H$_2$O, 0.41 mM CaC$\ell_2$·4H$_2$O, 0.82 mM MgSO$_4$·7H$_2$O, 10 $\mu$g/m$\ell$ penicillin sodium salt, and 10 $\mu$g/m$\ell$ streptomycin sulfate; pH 7.6). The aggregate was washed with the MBS three times and was treated in the MBS containing 0.2% (w/v) collagenase type II (available from Sigma Corp.) at room temperature for 1.5 hours. After the treatment, the aggregate of oocytes was washed with fresh MBS four or five times. Follicular cell membranes were removed from the oocyte surfaces with tweezers under the observation with a stereoscopic microscope. The prepared oocytes were immediately used for micro-injection.

(c) Expression of Synthesized RNA in Oocytes

50 n$\ell$ of the RNA synthesized in the step (a) were micro-injected to each oocyte prepared in the step (b). Thereafter, the oocytes were cultured in the MBS at 20°C for three days, thereby synthesizing a protein derived from the injected RNA.

Whether the human PAF receptor was contained in the protein synthesized in each oocyte was determined using a $Ca^{2+}$-sensitive C$\ell^-$ channel opening having ligand dependency as an index. This could be detected by measuring a change in membrane potential using the voltage clamp method.

A 0.5m$\ell$ tank was set under the stereoscopic microscope, and oocytes were placed while a Ringer's solution (115 mM NaC$\ell$, 2 mM KC$\ell$, 5 mM HEPES, 1.8 mM CaC$\ell_2$, and 1 mg/m$\ell$ BSA; pH 7.2) was perfused in the tank, and then the membrane potential of the oocytes were clamped. In this case, the command potential was set to be a reversal potential (normally -50 to -100 mV) or less of C$\ell^-$ in the Ringer's solution. While the membrane potential of the oocytes were kept clamped, the Ringer's solution containing a $10^{-7}$ M PAF was flowed. In an oocyte in which the PAF receptor was expressed, the PAF was bound to the receptor. Thus, a series of reactions occurred in an order of (activation of the GTP-binding protein) → (activation of phospholipase C) → (ascending of intracellular $Ca^{2+}$) → (opening of C$\ell^-$ channel), and an outward clamp current was obtained as a signal. Fig. 13 shows the response obtained when water was micro-injected, and Fig. 14 shows the response obtained when the RNA synthesized in the step (a) was micro-injected. Judging from the above results, it was confirmed that the cloned cDNA coded for the human PAF receptor.

(6) Construction of Expression Plasmid pEUKhPAF for COS Expression of Human PAF Receptor cDNA

An expression plasmid shown in Fig. 15 was constructed so as to introduce the cloned human PAF receptor cDNA into a cultured cell COS and to examine the presence/absence of expression.

A COS expression vector pEUK-C1 (available from Clonetec Corp.) was purchased from TOYOBO CO., LTD. This vector has SV 40 late promoter for initiating a transcription of an introduced cDNA and SV40 late polyadenilation signal and also has five cloning sites therebetween.

5 $\mu$g of the plasmid phPAF was digested by 5 units of BamHI and 5 units of XhoI, and the resultant reaction product was separated by electrophoresis using 1% agarose gel. About 1.7 Kbp of a DNA fragment containing the human RAF receptor cDNA in the agarose gel was recovered using Gene clean (available from Biotec Corp.) in accordance with the attached protocol. The DNA fragment was subjected to ethanol precipitation and was then dissolved in sterilized water.

5 $\mu$g of the expression vector pEUK-C1 was digested by 5 units of BamHI and 5 units of XhoI and then about 4.8 Kbp of a DNA fragment was recovered following the same way described above. The recovered DNA fragment was dissolved in sterilized water.

The two kinds of recovered DNA fragments, i.e., about 200 ng of the DNA fragment containing the human PAF receptor cDNA and about 50 ng of the vector DNA fragment were mixed and reacted to be bound. This reaction was performed using a TAKARA ligation kit in accordance with the attached protocol. By using this reaction solution, E.coli XL1-Blue was transformed by a conventional method to obtain a colony having resistance to Ampicillin (Amp$^R$). The plasmid DNA was recovered from this colony. The recovered plasmid was confirmed to be a target plasmid by agarose gel electrophoresis after this plasmid was digested by BamHI and XhoI. As described above, COS expression plasmid pEUKhPAF was constructed.

(7) Expression of Human PAF Receptor cDNA in COS7 Cell

COS expression plasmid pEUKhPAF constructed in (6) was introduced into a COS7 cell as a kind of a COS cell to examine the presence/absence of expression of the PAF receptor cDNA. The experiment was conducted in the following three steps.

(a) Introduction of Plasmid pEUKhPAF into COS7 Cells

COS 7 cells were cultured in a DMEM medium (available from Nissui Seiyaku KK) complemented with a 20% bovine fetal serum (available from Boehringer Mannheim Corp.), 0.6 g/$\ell$ L-glutamine, 1.5 g/$\ell$ sodium hydrogenecarbonate, 100 units/m$\ell$ penicillin, and 50 fg/m$\ell$ streptomycin at 37°C and in a condition of 5% $CO_2$. When the plasmid was introduced, the number of cells was adjusted in the order of $10^7$ cells/m$\ell$. A Selfect transfection kit available from Pharmacia Inc. was used to introduce the plasmid in accordance with the attached protocol. The cells introduced with the plasmid were kept cultured for three days to wait for expression of the introduced cDNA.

(b) Preparation of Membrane Fraction From Transformed Cell

After the culture, the transformed cells attached to the bottom surface of the culture vessel were recovered by sufficiently suspending the cells and centrifuging the cells at 800 × g and 4°C for 10 minutes. The precipitated cells were suspended in an HEPES buffer solution (25 mM HEPES, 10 mM MgC$\ell_2$, and 0.25 M sucrose; pH 7.4) cooled with ice, and were perfectly crushed by homogenizing the suspension on ice.

The resultant solution was centrifuged at 800 × g and 4°C for 10 minutes to obtain a supernatant, the supernatant was further centrifuged at 40,000 × g and 4°C for 20 minutes to obtain a precipitate, and the resultant precipitate was suspended in an HEPES buffer solution to obtain a membrane fraction subjected to a PAF receptor binding experiment.

(c) Confirmation of Human PAF Receptor Expressed on COS7 Cell

Whether the human PAF receptor was expressed on the transformed cell was confirmed by checking the presence of PAF receptor binding activity in the prepared membrane fraction. This experiment was conducted using PAF antagonist WEB2086 (available from NEN Corp.) labelled with tritium. To 100 $\mu\ell$ of a reaction buffer solution (25 mM HEPES, 10 mM MgC$\ell_2$, and 0.1% BSA; pH 7.4), 200 nM, 100 nM, 50 nM, 25 nM, 12.5 nM, 6.25 nM, or 3.125 mM [$^3$H] WEB2086 and 100 $\mu\ell$ of the membrane fraction prepared in the step (7)(b) were added and the resultant mixture was reacted at 25°C for 90 minutes. After this reaction, the reaction solution was immediately filtered through a glass filter (available from Wattman Corp.; GF/C) to cause the glass filter to adsorb the membrane fraction. The glass filter was sufficiently washed with a reaction buffer solution to remove a non-adsorbed [$^3$H] WEB2086. Thereafter, this glass filter was dried at 80°C for 30 minutes, and then added with liquid scintillator to measure a count. Note that a binding count in the presence of 10 $\mu\ell$ PAF in a reaction system at each point was also measured in order to detect nonspecific adsorption of the [$^3$H] WEB2086 to the membrane. Fig. 16 shows its result. Referring to Fig. 16, a specific bond is represented by o, and a non-specific bond is represented by •. It was therefore confirmed that the PAF receptor was expressed on the transformed cell membrane.

Industrial Applicability

A transformed cell produced using this cDNA can be applied to screening of an agonist and an antagonist, which future applications will be increasingly useful. In addition, this cDNA can also be applied to PAF quantitative assay which is currently difficult to achieve.

**Claims**

1. A cDNA for a guinea pig platelet- activating factor receptor having the following amino acid sequence:

Met Glu Leu Asn Ser Ser Ser Arg Val Asp
Ser Glu Phe Arg Tyr Thr Leu Phe Pro Ile
Val Tyr Ser Ile Ile Phe Val Leu Gly Ile
Ile Ala Asn Gly Try Val Leu Trp Val Phe
Ala Arg Leu Tyr Pro Ser Lys Lys Leu Asn
Glu Ile Lys Ile Phe Met Val Asn Leu Thr
Val Ala Asp Leu Leu Phe Leu Ile Thr Leu
Pro Leu Trp Ile Val Tyr Tyr Ser Asn Gln
Gly Asn Trp Phe Leu Pro Lys Phe Leu Cys
Asn Leu Ala Gly Cys Leu Phe Phe Ile Asn
Thr Tyr Cys Ser Val Ala Phe Leu Gly Val
Ile Thr Tyr Asn Arg Phe Gln Ala Val Lys
Tyr Pro Ile Lys Thr Ala Gln Ala Thr Thr
Arg Lys Arg Gly Ile Ala Leu Ser Leu Val
Ile Trp Val Ala Ile Val Ala Ala Ala Ser
Tyr Phe Leu Val Met Asp Ser Thr Asn Val
Val Ser Asn Lys Ala Gly Ser Gly Asn Ile
Thr Arg Cys Phe Glu His Tyr Glu Lys Gly
Ser Lys Pro Val Leu Ile Ile His Ile Cys
Ile Val Leu Gly Phe Phe Ile Val Phe Leu
Leu Ile Leu Phe Cys Asn Leu Val Ile Ile
His Thr Leu Leu Arg Gln Pro Val Lys Gln
Gln Arg Asn Ala Glu Val Arg Arg Arg Ala
Leu Trp Met Val Cys Thr Val Leu Ala Val
Phe Val Ile Cys Phe Val Pro His His Met
Val Gln Leu Pro Trp Thr Leu Ala Glu Leu
Gly Met Trp Pro Ser Ser Asn His Gln Ala
Ile Asn Asp Ala His Gln Val Thr Leu Cys
Leu Leu Ser Thr Asn Cys Val Leu Asp Pro
Val Ile Tyr Cys Phe Leu Thr Lys Lys Phe
Arg Lys His Leu Ser Glu Lys Leu Asn Ile
Met Arg Ser Ser Gln Lys Cys Ser Arg Val


Thr Thr Asp Thr Gly Thr Glu Met Ala Ile
Pro Ile Asn His Thr Pro Val Asn Pro Ile
Lys Asn.


2. A cDNA for a guinea pig platelet-activating factor receptor according to claim 1, said cDNA having the

following base sequence:

```
ATG GAG TTA AAC AGC TCT TCC CGG GTG GAT
TCT GAG TTT CGA TAC ACA CTC TTC CCA ATT
GTT TAC AGC ATT ATC TTT GTG CTG GGG ATC
ATC GCC AAT GGC TAT GTG CTA TGG GTC TTT
GCC CGC CTA TAC CCT TCC AAG AAA CTA AAT
GAG ATA AAG ATC TTC ATG GTG AAC CTC ACG
GTG GCT GAC CTG CTC TTC CTG ATC ACC TTG
CCT CTG TGG ATC GTC TAC TAT TCC AAC CAG
GGC AAC TGG TTT CTA CCC AAA TTC CTT TGT
AAC CTG GCT GGC TGC CTC TTC TTC ATC AAC
ACC TAC TGC TCT GTG GCC TTC CTG GGA GTG
ATC ACC TAT AAC CGC TTC CAG GCA GTG AAA
TAT CCC ATC AAG ACT GCT CAG GCC ACC ACC
CGC AAG CGA GGC ATC GCT TTG TCC TTG GTC
ATC TGG GTG GCC ATT GTG GCT GCT GCA TCT
TAC TTC CTT GTC ATG GAC TCC ACC AAC GTA
GTG TCC AAT AAG GCA GGC TCG GGC AAC ATT
ACT CGT TGC TTT GAG CAC TAT GAG AAG GGC
AGC AAG CCT GTC CTC ATC ATC CAT AGC TGC
ATT GTG CTC GGC TTC TTC ATT GTC TTC CTT
CTC ATC CTC TTC TGC AAC CTG GTC ATC ATC
CAC ACG CTG CTC AGG CAG CCA GTG AAG CAG
CAG CGC AAT GCT GAG GTC AGG CGC CGA GCG
CTG TGG ATG GTT TGC ACG GTT CTG GCA GTG
TTT GTC ATC TGC TTT GTA CCA CAC CAC ATG
GTA CAG CTG CCC TGG ACC CTG GCT GAG CTG
GGG ATG TGG CCA AGC AGC AAC CAC CAG GCT
ATT AAT GAT GCA CAT CAG GTC ACC CTC TGC
CTC CTT AGC ACC AAC TGT GTC TTA GAC CCT
GTC ATC TAC TGT TTC CTC ACC AAG AAG TTC
CGC AAG CAC CTC ACG CAA AAG CTT AAT ATC


ATG CGC AGC AGC CAG AAA TGC TCC CGG GTC
ACC ACT GAC ACA GGC ACT GAA ATG GCC ATT
CCA ATC AAC CAC ACT CCT GTC AAT CCC ATC
AAA AAT NNN
```

wherein NNN represents TGA, TAA or TAG.

3. A cDNA for a human platelet-activating factor receptor having the following amino acid sequence:

```
Met Glu Pro His Asp Ser Ser His Met Asp
Ser Glu Phe Arg Tyr Thr Leu Phe Pro Ile
Val Tyr Ser Ile Ile Phe Val Leu Gly Val
Ile Ala Asn Gly Tyr Val Leu Trp Val Phe
Ala Arg Leu Tyr Pro Cys Lys Lys Phe Asn
Glu Ile Lys Ile Phe Met Val Asn Leu Thr
Met Ala Asp Met Leu Phe Leu Ile Thr Leu
Pro Leu Trp Ile Val Tyr Tyr Gln Asn Gln
Gly Asn Trp Ile Leu Pro Lys Phe Leu Cys
Asn Val Ala Gly Cys Leu Phe Phe Ile Asn
Thr Tyr Cys Ser Val Ala Phe Leu Gly Val
Ile Thr Tyr Asn Arg Phe Gln Ala Val Thr
Arg Pro Ile Lys Thr Ala Gln Ala Asn Thr
Arg Lys Arg Gly Ile Ser Leu Ser Leu Val
Ile Trp Val Ala Ile Val Gly Ala Ala Ser
Tyr Phe Leu Ile Leu Asp Ser Thr Asn Thr
Val Pro Asp Ser Ala Gly Ser Gly Asn Val
Thr Arg Cys Phe Glu His Tyr Glu Lys Gly
Ser Val Pro Val Leu Ile Ile His Ile Phe
Ile Val Phe Ser Phe Phe Leu Val Phe Leu
Ile Ile Leu Phe Cys Asn Leu Val Ile Ile
Arg Thr Leu Leu Met Gln Pro Val Gln Gln
Gln Arg Asn Ala Glu Val Lys Arg Arg Ala
Leu Trp Met Val Cys Thr Val Leu Ala Val
Phe Ile Ile Cys Phe Val Pro His His Val
Val Gln Leu Pro Trp Thr Leu Ala Glu Leu
Gly Phe Gln Asp Ser Lys Phe His Gln Ala
Ile Asn Asp Ala His Gln Val Thr Leu Cys
Leu Leu Ser Thr Asn Cys Val Leu Asp Pro
Val Ile Tyr Cys Phe Leu Thr Lys Lys Phe
```

```
Arg Lys His Leu Thr Glu Lys Phe Tyr Ser
Met Arg Ser Ser Arg Lys Cys Ser Arg Ala
Thr Thr Asp Thr Val Thr Glu Val Val Val
Pro Phe Asn Gln Ile Pro Gly Asn Ser Leu
Lys Asn.
```

4. A cDNA for a human platelet-activating factor receptor according to claim 3, said cDNA having the following base sequence:

```
ATG GAG CCA CAT GAC TCC TCC CAC ATG GAC
TCT GAG TTC CGA TAC ACT CTC TTC CCG ATT
GTT TAC AGC ATC ATC TTT GTG CTC GGG GTC
ATT GCT AAT GGC TAC GTG CTG TGG GTC TTT
GCC CGC CTG TAC CCT TGC AAG AAA TTC AAT
GAG ATA AAG ATC TTC ATG GTG AAC CTC ACC
ATG GCG GAC ATG CTC TTC TTG ATC ACC CTG
CCA CTT TGG ATT GTC TAC TAC CAA AAC CAG
GGC AAC TGG ATA CTC CCC AAA TTC CTG TGC
AAC GTG GCT GGC TGC CTT TTC TTC ATC AAC
ACC TAC TGC TCT GTG GCC TTC CTG GGC GTC
ATC ACT TAT AAC CGC TTC CAG GCA GTA ACT
CGG CCC ATC AAG ACT GCT CAG GCC AAC ACC
CGC AAG CGT GGC ATC TCT TTG TCC TTG GTC
ATC TGG GTG GCC ATT GTG GGA GCT GCA TCC
TAC TTC CTC ATC CTG GAC TCC ACC AAC ACA
GTG CCC GAC AGT GCT GGC TCA GGC AAC GTC
ACT CGC TGC TTT GAG CAT TAC GAG AAG GGC
AGC GTG CCA GTC CTC ATC ATC CAC ATC TTC
ATC GTG TTC AGC TTC TTC CTG GTC TTC CTC
ATC ATC CTC TTC TGC AAC CTG GTC ATC ATC
CGT ACC TTG CTC ATG CAG CCG GTG CAG CAG
CAG CGC AAC GCT GAA GTC AAG CGC CGG GCG
CTG TGG ATG GTG TGC ACG GTC TTG GCG GTG
TTC ATC ATC TGC TTC GTG CCC CAC CAC GTG
GTG CAG CTG CCC TGG ACC CTT GCT GAG CTG
GGC TTC CAG GAC AGC AAA TTC CAC CAG GCC
ATT AAT GAT GCA CAT CAG GTC ACC CTC TGC
CTC CTT AGC ACC AAC TGT GTC TTA GAC CCT
```

```
GTT ATC TAC TGT TTC CTC ACC AAG AAG TTC
CGC AAG CAC CTC ACC GAA AAG TTC TAC AGC
ATG CGC AGT AGC CGG AAA TGC TCC CGG GCC
ACC ACG GAT ACG GTC ACT GAA GTG GTT GTG
CCA TTC AAC CAG ATC CCT GGC AAT TCC CTC
AAA AAT NNN
```

wherein NNN represents TGA, TAA or TAG.

5. A recombinant DNA containing said cDNA for the human platelet-activating receptor of claim 3, and is capable of self-propagation in microorganism cells.

6. A recombinant DNA according to claim 5, in which said cDNA for human platelet-activating factor receptor has the following base sequence:

```
ATG GAG CCA CAT GAC TCC TCC CAC ATG GAC
TCT GAG TTC CGA TAC ACT CTC TTC CCG ATT
GTT TAC AGC ATC ATC TTT GTG CTC GGG GTC
ATT GCT AAT GGC TAC GTG CTG TGG GTC TTT
GCC CGC CTG TAC CCT TGC AAG AAA TTC AAT
GAG ATA AAG ATC TTC ATG GTG AAC CTC ACC
ATG GCG GAC ATG CTC TTC TTG ATC ACC CTG
CCA CTT TGG ATT GTC TAC TAC CAA AAC CAG
GGC AAC TGG ATA CTC CCC AAA TTC CTG TGC
AAC GTG GCT GGC TGC CTT TTC TTC ATC AAC
ACC TAC TGC TCT GTG GCC TTC CTG GGC GTC
ATC ACT TAT AAC CGC TTC CAG GCA GTA ACT
CGG CCC ATC AAG ACT GCT CAG GCC AAC ACC
CGC AAG CGT GGC ATC TCT TTG TCC TTG GTC
ATC TGG GTG GCC ATT GTG GGA GCT GCA TCC
TAC TTC CTC ATC CTG GAC TCC ACC AAC ACA
GTG CCC GAC AGT GCT GGC TCA GGC AAC GTC
ACT CGC TGC TTT GAG CAT TAC GAG AAG GGC
AGC GTG CCA GTC CTC ATC ATC CAC ATC TTC
ATC GTG TTC AGC TTC TTC CTG GTC TTC CTC
ATC ATC CTC TTC TGC AAC CTG GTC ATC ATC
CGT ACC TTG CTC ATG CAG CCG GTG CAG CAG
CAG CGC AAC GCT GAA GTC AAG CGC CGG GCG
CTG TGG ATG GTG TGC ACG GTC TTG GCG GTG
```

```
TTC ATC ATC TGC TTC GTG CCC CAC CAC GTG
GTG CAG CTG CCC TGG ACC CTT GCT GAG CTG
GGC TTC CAG GAC AGC AAA TTC CAC CAG GCC
ATT AAT GAT GCA CAT CAG GTC ACC CTC TGC
CTC CTT AGC ACC AAC TGT GTC TTA GAC CCT
GTT ATC TAC TGT TTC CTC ACC AAG AAG TTC
CGC AAG CAC CTC ACC GAA AAG TTC TAC AGC
ATG CGC AGT AGC CGG AAA TGC TCC CGG GCC
ACC ACG GAT ACG GTC ACT GAA GTG GTT GTG
CCA TTC AAC CAG ATC CCT GGC AAT TCC CTC
AAA AAT NNN
```

wherein NNN represents TGA, TAA or TAG.

**7.** A transformed cell which contains said DNA of claim 6.

Met Glu Leu Asn Ser Ser Ser Arg Val Asp Ser Glu Phe Arg Tyr Thr Leu Phe Pro Ile
1               5                   10                  15                  20

Val Tyr Ser Ile Ile Phe Val Leu Gly Ile Ile Ala Asn Gly Try Val Leu Trp Val Phe
                25                  30                  35                  40

Ala Arg Leu Tyr Pro Ser Lys Lys Leu Asn Glu Ile Lys Ile Phe Met Val Asn Leu Thr
                45                  50                  55                  60

Val Ala Asp Leu Leu Phe Leu Ile Thr Leu Pro Leu Trp Ile Val Tyr Tyr Ser Asn Gln
                65                  70                  75                  80

Gly Asn Trp Phe Leu Pro Lys Phe Leu Cys Asn Leu Ala Gly Cys Leu Phe Phe Ile Asn
                85                  90                  95                  100

Thr Tyr Cys Ser Val Ala Phe Leu Gly Val Ile Thr Tyr Asn Arg Phe Gln Ala Val Lys
                105                 110                 115                 120

Tyr Pro Ile Lys Thr Ala Gln Ala Thr Thr Arg Lys Arg Gly Ile Ala Leu Ser Leu Val
                125                 130                 135                 140

Ile Trp Val Ala Ile Val Ala Ala Ala Ser Tyr Phe Leu Val Met Asp Ser Thr Asn Val
                145                 150                 155                 160

Val Ser Asn Lys Ala Gly Ser Gly Asn Ile Thr Arg Cys Phe Glu His Tyr Glu Lys Gly
                165                 170                 175                 180

Ser Lys Pro Val Leu Ile Ile His Ile Cys Ile Val Leu Gly Phe Phe Ile Val Phe Leu
                185                 190                 195                 200

Leu Ile Leu Phe Cys Asn Leu Val Ile Ile His Thr Leu Leu Arg Gln Pro Val Lys Gln
                205                 210                 215                 220

Gln Arg Asn Ala Glu Val Arg Arg Arg Ala Leu Trp Met Val Cys Thr Val Leu Ala Val
                225                 230                 235                 240

Phe Val Ile Cys Phe Val Pro His His Met Val Gln Leu Pro Trp Thr Leu Ala Glu Leu
                245                 250                 255                 260

Gly Met Trp Pro Ser Ser Asn His Gln Ala Ile Asn Asp Ala His Gln Val Thr Leu Cys
                265                 270                 275                 280

Leu Leu Ser Thr Asn Cys Val Leu Asp Pro Val Ile Tyr Cys Phe Leu Thr Lys Lys Phe
                285                 290                 295                 300

Arg Lys His Leu Ser Glu Lys Leu Asn Ile Met Arg Ser Ser Gln Lys Cys Ser Arg Val
                305                 310                 315                 320

Thr Thr Asp Thr Gly Thr Glu Met Ala Ile Pro Ile Asn His Thr Pro Val Asn Pro Ile
                325                 330                 335                 340

Lys Asn

F I G. 1

```
ATG GAG TTA AAC AGC TCT TCC CGG GTG GAT TCT GAG TTT CGA TAC ACA CTC TTC CCA ATT
GTT TAC AGC ATT ATC TTT GTG CTG GGG ATC ATC GCC AAT GGC TAT GTG CTA TGG GTC TTT
GCC CGC CTA TAC CCT TCC AAG AAA CTA AAT GAG ATA AAG ATC TTC ATG GTG AAC CTC ACG
GTG GCT GAC CTG CTC TTC CTG ATC ACC TTG CCT CTG TGG ATC GTC TAC TAT TCC AAC CAG
GGC AAC TGG TTT CTA CCC AAA TTC CTT TGT AAC CTG GCT GGC TGC CTC TTC TTC ATC AAC
ACC TAC TGC TCT GTG GCC TTC CTG GGA GTG ATC ACC TAT AAC CGC TTC CAG GCA GTG AAA
TAT CCC ATC AAG ACT GCT CAG GCC ACC ACC CGC AAG CGA GGC ATC GCT TTG TCC TTG GTC
ATC TGG GTG GCC ATT GTG GCT GCT GCA TCT TAC TTC CTT GTC ATG GAC TCC ACC AAC GTA
GTG TCC AAT AAG GCA GGC TCG GGC AAC ATT ACT CGT TGC TTT GAG CAC TAT GAG AAG GGC
AGC AAG CCT GTC CTC ATC ATC CAT AGC TGC ATT GTG CTC GGC TTC TTC ATT GTC TTC CTT
CTC ATC CTC TTC TGC AAC CTG GTC ATC ATC CAC ACG CTG CTC AGG CAG CCA GTG AAG CAG
CAG CGC AAT GCT GAG GTC AGG CGC CGA GCG CTG TGG ATG GTT TGC ACG GTT CTG GCA GTG
TTT GTC ATC TGC TTT GTA CCA CAC CAC ATG GTA CAG CTG CCC TGG ACC CTG GCT GAG CTG
GGG ATG TGG CCA AGC AGC AAC CAC CAG GCT ATT AAT GAT GCA CAT CAG GTC ACC CTC TGC
CTC CTT AGC ACC AAC TGT GTC TTA GAC CCT GTC ATC TAC TGT TTC CTC ACC AAG AAG TTC
CGC AAG CAC CTC ACG CAA AAG CTT AAT ATC ATG CGC AGC AGC CAG AAA TGC TCC CGG GTC
ACC ACT GAC ACA GGC ACT GAA ATG GCC ATT CCA ATC AAC CAC ACT CCT GTC AAT CCC ATC
AAA AAT NNN
```

F I G.  2

```
GGCACGAGTG CGGCCAGGCT GGACCCAGGG ACACAATAGA CACTACAGCC AAGCCACTGC
CGCTGCTGCA GGCACTGCCA GGACCCTATG AGGAGCTGAC TCTTGCAGCC CAGAGCC
```

F I G.  3

```
TTCCTGCTTG CTGGGGTCAG GCCCAAAGTC TTCTGTTCCA TAAGGGGTAT CTGCTGTAGT
CACCTCCTCC CTGGCACTGG TGGCCCATGA AGTATAAAGC CTACCTCACA CACAGGCAGG
GACAATGGCC ATGCTGGACT GCTAGAAAAT CCAGAACTTG AACGAGTACC TTCACCCCCT
TTGCACACAT CTATGACCTG ATCCTGAGCC TCTGGGTCCG ATGAAGCTAA GAGGAATCTG
AGGGTCAGGG GACAGACTCT GAGCCATTCC CAGGTTGCCT ACTAGGGTAG TAGCCTAAAG
CCCTTAGGCA ATGACTCCCA AGTGCTCAGA CAGTGGAAGT GGCCTGGTAG AATCTTCTGA
TTCCGCCAGC AAACCTGTCC CAAATGACAA CTACAGAAAA GAAAACATGA ACAGAGCAAA
AGCTAAGACA TGAGCTTGTT TCCTGCACAG GATTTGGCTC AAGCTTGGGT CAGTCCTGAG
GGGCTGGGAG AGAGAGAGCC TCTCCTTCGT TTTCCAGCCT TACTAACAGA CCCTGAGTGA
AGACCTTCAG TGGGGCAGAA CGTCTCTTTA AAGCTTTTCC CGAGAGCACA CCAGCCAGGG
CAAGTCCAGA GGCTTCCATG GGAACAGCGC CTACTCAGGA AAGAGCCTGA GGTTTCTGGT
TGGGAAAAAT CTGCTCCTTC TCTGGAAGGC CCTCTCTGCC TAATTTCCAG GAAATAGAAG
GGACAGTTAC ACAGGCTGGT GGGTGTCAGC TTCTCCAGTA TTTCTAAGAC AGCTGCCTGT
CATTTTTGAT AAGCATTCAA ATGAGACGCT CTCCTTCCTT TAGCCAAGAA CTTGCAGTGT
GAGCCCTGGC CCTCAGACAT GGGGTGTCCA GTGCTCCTCT GAGAACTTCC TTCCAGCCAT
TGTCGCACTA ATTTGGGCTT TTCTGCCTGC CAGGCAACAC TGGATTGCTA ATTTTGATAC
ATAGTTCCCC AGGGGCTAGT AACCTGCTTG GGTCTGGGAG AGTAGCTGTG CACTGGCTCT
AGCTGATGGT GACCGTACCC GGTGGCCAGA GCAGCACACT CAAGCGTTGG TGTCACACAG
ACCCAAGTTC AGTCTTGGCT TTGTCACTTC CTGCCGAGTG GCTGGGGACT AATTTTCTAC
CTCTTAGTGT TGTTGCCTGT GAGGTAAAAC CTACCTCCCT GAGGTGTTCG CGCAGCAAAT
GCCGAGAAAC GCACCTCTTG GTCAGGGTGA CTGCATCCCA GACCCTCCCT TTGACCTGAG
AATGTGGACC CTTCAGTCCT CCGGTAGAGG CTGAAACAGT AAAGGGTGGC ACCTGGATGT
GGGGATGCTG CTTCAGGCTT TGCAGACCAC TCAGGTAAGA TATATGGACT CTCGAACTCT
GAGAGAGCGA CAGAGACAGA CACAGACACA GACACAGAGA CAGAGAGACA GAGACAGAGA
GGCAGAGAGA GAAGGGGGAG TGGGGTGGGT GTGTGCAGGA GTACTGCCCT GCGTGATTGT
GATTAGAGAA AATGACTCTG CTCACAGAAT AAATTTTCTA GAGCAAAGCA GAAGGCAGGG
AGAAATGACA GGAGCATGGT GGGAAGAGGG GACAGATCTT GCAGGGGCCT GCTGGGTGAC
TGTCCTCTAC ACGCCTTCCA TTGTGTCCCA TGAGAGTCTC TTGTCCGGAC ACCTTACTTA
GTTGCTTAAG CTGATTTGAA TGAGTGTCTA TTACTGGCAG CCCGCAGAGC AGCAGATAAA
GAGTAAGTCT ATGTGCTAAC ATTGCACAGT GTTTCCTGAA CATCAGCCAT TTGCATCTCT
CCTTGCAAGC TTTGTAATGT CCACGCACCG GCTGTGTGTT TACTTAATAA ATACTTTTTC
TTTAAATCCA A
```

F I G. 4

```
Met Glu Pro His Asp Ser Ser His Met Asp Ser Glu Phe Arg Tyr Thr Leu Phe Pro Ile
 1               5                   10                  15                  20
Val Tyr Ser Ile Ile Phe Val Leu Gly Val Ile Ala Asn Gly Tyr Val Leu Trp Val Phe
                25                  30                  35                  40
Ala Arg Leu Tyr Pro Cys Lys Lys Phe Asn Glu Ile Lys Ile Phe Met Val Asn Leu Thr
                45                  50                  55                  60
Met Ala Asp Met Leu Phe Leu Ile Thr Leu Pro Leu Trp Ile Val Tyr Tyr Gln Asn Gln
                65                  70                  75                  80
Gly Asn Trp Ile Leu Pro Lys Phe Leu Cys Asn Val Ala Gly Cys Leu Phe Phe Ile Asn
                85                  90                  95                  100
Thr Tyr Cys Ser Val Ala Phe Leu Gly Val Ile Thr Tyr Asn Arg Phe Gln Ala Val Thr
                105                 110                 115                 120
Arg Pro Ile Lys Thr Ala Gln Ala Asn Thr Arg Lys Arg Gly Ile Ser Leu Ser Leu Val
                125                 130                 135                 140
Ile Trp Val Ala Ile Val Gly Ala Ala Ser Tyr Phe Leu Ile Leu Asp Ser Thr Asn Thr
                145                 150                 155                 160
Val Pro Asp Ser Ala Gly Ser Gly Asn Val Thr Arg Cys Phe Glu His Tyr Glu Lys Gly
                165                 170                 175                 180
Ser Val Pro Val Leu Ile Ile His Ile Phe Ile Val Phe Ser Phe Phe Leu Val Phe Leu
                185                 190                 195                 200
Ile Ile Leu Phe Cys Asn Leu Val Ile Ile Arg Thr Leu Leu Met Gln Pro Val Gln Gln
                205                 210                 215                 220
Gln Arg Asn Ala Glu Val Lys Arg Arg Ala Leu Trp Met Val Cys Thr Val Leu Ala Val
                225                 230                 235                 240
Phe Ile Ile Cys Phe Val Pro His His Val Val Gln Leu Pro Trp Thr Leu Ala Glu Leu
                245                 250                 255                 260
Gly Phe Gln Asp Ser Lys Phe His Gln Ala Ile Asn Asp Ala His Gln Val Thr Leu Cys
                265                 270                 275                 280
Leu Leu Ser Thr Asn Cys Val Leu Asp Pro Val Ile Tyr Cys Phe Leu Thr Lys Lys Phe
                285                 290                 295                 300
Arg Lys His Leu Thr Glu Lys Phe Tyr Ser Met Arg Ser Ser Arg Lys Cys Ser Arg Ala
                305                 310                 315                 320
Thr Thr Asp Thr Val Thr Glu Val Val Val Pro Phe Asn Gln Ile Pro Gly Asn Ser Leu
                325                 330                 335                 340
Lys Asn
```

# F I G. 5

```
ATG GAG CCA CAT GAC TCC TCC CAC ATG GAC TCT GAG TTC CGA TAC ACT CTC TTC CCG ATT
GTT TAC AGC ATC ATC TTT GTG CTC GGG GTC ATT GCT AAT GGC TAC GTG CTG TGG GTC TTT
GCC CGC CTG TAC CCT TGC AAG AAA TTC AAT GAG ATA AAG ATC TTC ATG GTG AAC CTC ACC
ATG GCG GAC ATG CTC TTC TTG ATC ACC CTG CCA CTT TGG ATT GTC TAC TAC CAA AAC CAG
GGC AAC TGG ATA CTC CCC AAA TTC CTG TGC AAC GTG GCT GGC TGC CTT TTC TTC ATC AAC
ACC TAC TGC TCT GTG GCC TTC CTG GGC GTC ATC ACT TAT AAC CGC TTC CAG GCA GTA ACT
CGG CCC ATC AAG ACT GCT CAG GCC AAC ACC CGC AAG CGT GGC ATC TCT TTG TCC TTG GTC
ATC TGG GTG GCC ATT GTG GGA GCT GCA TCC TAC TTC CTC ATC CTG GAC TCC ACC AAC ACA
GTG CCC GAC AGT GCT GGC TCA GGC AAC GTC ACT CGC TGC TTT GAG CAT TAC GAG AAG GGC
AGC GTG CCA GTC CTC ATC ATC CAC ATC TTC ATC GTG TTC AGC TTC TTC CTG GTC TTC CTC
ATC ATC CTC TTC TGC AAC CTG GTC ATC ATC CGT ACC TTG CTC ATG CAG CCG GTG CAG CAG
CAG CGC AAC GCT GAA GTC AAG CGC CGG GCG CTG TGG ATG GTG TGC ACG GTC TTG GCG GTG
TTC ATC ATC TGC TTC GTG CCC CAC CAC GTG GTG CAG CTG CCC TGG ACC CTT GCT GAG CTG
GGC TTC CAG GAC AGC AAA TTC CAC CAG GCC ATT AAT GAT GCA CAT CAG GTC ACC CTC TGC
CTC CTT AGC ACC AAC TGT GTC TTA GAC CCT GTT ATC TAC TGT TTC CTC ACC AAG AAG TTC
CGC AAG CAC CTC ACC GAA AAG TTC TAC AGC ATG CGC AGT AGC CGG AAA TGC TCC CGG GCC
ACC ACG GAT ACG GTC ACT GAA GTG GTT GTG CCA TTC AAC CAG ATC CCT GGC AAT TCC CTC
AAA AAT NNN
```

F I G.  6

```
TTCACGAGGG CTGGGGCCAG GACCCAGACA GAGACACACG GTCACTGCAG CTGAAGCCGC
TGCCCCTGCT ACAGGCACCA CCAGGACCAG CTGATCATTC CAGCCCACAG CA
```

F I G.  7

TCCCTGCTTC CAGGCCTGAA GTCTTCTCCT CCATGAAACA TCATGACTGA GCTGGGGGAA

GAAGGGATAT CTACTGTGGG TCTGGGCACC ACCTCTGTGG CACTGGTGGG CCATTAGATT

TGGAGGCTAC CTCACCTGGG CAGGGATGAT GCAGAGCCAG GCTGTTGGAA AATCCAGAAC

TCAAATGAGC CCCTTCATCC GCCTGTGGGC GCATACTACA GTAACTGTGA CTGATGACTT

TATCCTGAGT CCCTTAATCT TATGGGGCCG GAAGGAATGT CAGGGCCAGG TGCAGACCTT

GGGGGAAGAC TTTAAACCAC CTAGTTCTCC CACTGGGGCA TCGGTCTAAA GCTTTGGGGG

AGTGGCCCCA GTGGCTCACA CCTGTAATCC CAGCACTTTG GGAGGCCGAG GTGGGCAGAT

CATGGGTCAA GAGATCGAGA CATCCTGGCC AACATTGTAA AACCCCATCT CTACTAAAAC

ATACAAAAAT TAGCCGGGCA TGGTGCACAC GCCTGTAGTC CCAGCTACTC AGGAGGCTGA

GGCAGGAGAA TCGCTTGAAC CTGGGAGGCA GAGGTTGCAG TGAACCTAGA TTGCACCATT

GCACTCTAGC CTGGCAACAG AGGCAGATTC CCTCCTGCC

# F I G. 8

FIG. 9

$10^{-7}$ M PAF

100nA

120 sec

FIG. 10

$10^{-8}$ M lyso-PAF      $10^{-8}$ M PAF

400nA

120 sec

# FIG. 11

Zp74
6Kbp

Amp$^R$

T7 プロモーター

Xho I

Bam HI

PAF 受容体cDNA

Xho I, Bam HI 消化反応

Xho I ——— Bam HI

PAF 受容体cDNA

pEUK-C1
4.8 Kbp

Amp$^R$

SV40 後期 プロモーター

SV40 後期アデニレーションシグナル

Xho I   Bam HI

Xho I, Bam HI 消化反応

Amp$^R$

Xho I   Bam HI

ライゲーション反応

pEUKPAF
7.8 kbp

Xho I

Bam HI

F I G . 1 2

F I G . 1 3

PAF ($10^{-7}$M)

50nA

1min

F I G . 1 4

PAF ($10^{-7}$M)

100nA

1min

FIG. 15

phPAF
4.7 k b p
Amp^R
T7 プロモーター
Xho I
Bam HI
hPAF受容体cDNA

↓

Xho I, Bam HI 消化反応

↓

Xho I —— hPAF受容体 cDNA —— Bam HI

pEUK-C1
4.8 k b p
Amp^R
SV40 後期 プロモーター
SV40 後期ポリ アデニレーション シグナル
Xho I    Bam HI

↓

Xho I, Bam HI 消化反応

↓

Amp
Xho I    Bam HI

ライゲーション反応

↓

pEUKhPAF
6.5 k b p
Amp^R
Xho I
Bam HI

FIG. 16

# INTERNATIONAL SEARCH REPORT

International Application No  PCT/JP91/01779

**I. CLASSIFICATION OF SUBJECT MATTER (if several classification symbols apply, indicate all) ⁶**

According to International Patent Classification (IPC) or to both National Classification and IPC

Int. Cl⁵  C12N15/10, C12N5/16//C12P21/02

**II. FIELDS SEARCHED**

| Minimum Documentation Searched ⁷ | |
|---|---|
| Classification System | Classification Symbols |
| IPC | C12N15/00-15/90 |

Documentation Searched other than Minimum Documentation
to the Extent that such Documents are Included in the Fields Searched ⁸

Biosis Database, Chemical Abstract Database (CA, REG)

**III. DOCUMENTS CONSIDERED TO BE RELEVANT ⁹**

| Category * | Citation of Document, ¹¹ with indication, where appropriate, of the relevant passages ¹² | Relevant to Claim No. ¹³ |
|---|---|---|
| A | J. Lipid, Res., Vol. 28, No. 6, 1987, Bittman R. et al. "Synthesis and biochemical studies of analogs of platelet-activating-factor bearing a methyl group at C2 of the glycerol backbone"  p. 733-738 | 1-7 |
| A | Int. J. Tissue. React., Vol. 7, No. 5, 1985, Sanchez Crespo M. et al. "Synthesis of platelet-activating-factor from human polymorphonuclear leukocytes regulation and pharmacological approaches" p. 345-350 | 1-7 |

* Special categories of cited documents: ¹⁰

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| March 25, 1992 (25. 03. 92) | April 7, 1992 (07. 04. 92) |
| International Searching Authority | Signature of Authorized Officer |
| Japanese Patent Office | |

Form PCT/ISA/210 (second sheet) (January 1985)